# EUROPEAN PATENT SPECIFICATION

(11) **EP 2 692 730 B1**
(45) Date of publication and mention of the grant of the patent: **14.11.2018**
(21) Application number: 12765410.1
(22) Date of filing: 05.03.2012
(51) Int. Cl.: C07K 1/06, C07K 14/575, A61K 38/22, C07K 14/605, A61P 3/10, A61K 38/00, A61K 47/60

(54) **SITE-DIRECTED MONO-SUBSTITUTED PEGYLATED EXENDIN ANALOG AND PREPARATION METHOD THEREFOR**
ORTSGERICHTETE MONOSUBSTITUIERTE PEGYLIERTE EXENDIN-ANALOGA UND HERSTELLUNGSVERFAHREN DAFÜR
ANALOGUE D'EXENDINE PEGYLÉ MONOSUBSTITUÉ DIRIGÉ ET SON PROCÉDÉ DE PRÉPARATION

(30) Priority: 30.03.2011 CN 201110078314
(43) Date of publication of application: 05.02.2014
(73) Proprietor: Shanghai Benemae Pharmaceutical Corporation, Zhoupu Town Pudong New Area Shanghai (CN)
(72) Inventor: YUE, Peng, Shanghai 200235 (CN)
(74) Representative: Gulde & Partner
(86) International application number: PCT/CN2012/071910
(87) International publication number: WO 2012/130015

(56) References cited:
- WO-A1-2007/061148
- WO-A1-2007/061148
- WO-A1-2008/058461
- WO-A1-2008/130066
- WO-A2-2010/107256
- CN-A- 101 125 207

## Description

### Summary

The present invention relates to site-specific mono-PEGylated Exendin analogs, the preparation method and pharmaceutical use.

### Background

In the 1960s, McIntyre and Elrick et al. discovered the 'incretin effect' that oral glucose administration induced much higher insulin response than intravenous infusion. Further studies conducted by Perley et al. demonstrated that the 'incretin effect' comprised over 50% of the postprandial insulin release. In 1986, Nauck et al. found the reduction of 'incretin effect' in patients with type 2 diabetes mellitus (T2DM), which indicated that incretin system abnormality might be one of the pathogenesis of T2DM.

Incretins are gut-derived and glucose level-dependent hormones, including GLP-1 and glucose-dependent insulinotropic peptide (GIP), can stimulate insulin secretion after meals. GLP-1 appears to play a more important role in the development of therapeutic strategies for T2DM. GLP-1 promotes the insulin secretion to control the blood sugar, simultaneously, it stimulates islet β cell proliferation, prevents β cell apoptosis, inhibits glucagon release, gastrointestinal motility and gastric secretion, delays gastric emptying, produces satiety and suppresses appetite. However, GLP-1 is degraded by DPP-IV rapidly in vivo, having a short half-life of 1-2 minutes. Consequently, its application and development is limited.

Exendin-4, isolated from the saliva of the lizard Heloderma suspectum, is a GLP-1 analog with 39 amino acid, sharing 53% homology with GLP-1. Similar to GLP-1, it can bind to the GLP-1 receptor, stimulating insulin secretion, reducing fasting and postprandial blood glucose with the purpose of treating T2DM. N-terminal second amino acid residue of GLP-1 is alanine which is replaced by glycine in Exendin-4. This renders the resistance to degradation by dipeptidase (DPP-IV) in vivo, prolonging the half-life from 1-2 min to 2-4h. Therefore, it has been developed into listed products, administered twice daily (Byetta, Amylin Corporation).

However, frequent injections result in poor patient compliance, arousing the unstoppable research on sustained release dosage. For instance, liraglutide (NN-2211) is developed by Novo Nordisk and is administered once daily; Exenatide LAR (PLGA microspheres) is developed by Amylin and is administered once weekly. Therefore, long-acting formulations or non-injection formulations becomes the trend of research and development

The most widely studied and used pharmaceutical formulation techniques for long-acting therapeutic peptide/protein is PEGylation. Activated polyethylene glycol (PEG) derivatives react with specific accessible amino acid residues on therapeutic proteins to form new molecules. The most fully studied and widely used medical long-acting peptide protein technology is pegylation technology, through which new molecules can be formed by the reaction between activated polyethylene glycol reagent and specific amino acid residues on the peptide or protein, Polyethylene glycol as a kind of watersoluble macromolecular may have given the following features to the protein/peptide: (1) an improvement in drug solubility; (2) a decrease in immunogenicity; (3) an extension of half-life in blood due to the reduced kidney clearance. These features contribute to the prolonging drug action and the improving patient compliance.

PEGylation technology generally allows activated PEG derivatives to react with active residues (by order of activity, mercapto, amino, carboxy, etc.) on peptide or protein, forming PEGylated product with a stable chemical bond. However, the existence of multiple reactive residues results in a mixture of various mono-PEGylated products with different residues PEGylated. Moreover, in condition of high molar ratio of PEG and continuous reaction, multi-PEGylated compounds may be formed and that can significantly affect drug activity and efficacy in vivo. In addition, quality controls of multi-PEGylated products are difficult. Therefore, the PEGylated listed drugs are basically mono-PEGylated.

Since the position of the mono-PEGylation affects the activity of the peptide or protein drugs significantly, preparing mono-PEGylated product with the best optional active site is necessary.

### The existing technology of site-specific PEGylation comprises two types:

The first is to control number of reactive residues on the structure. The widely used approach is to mutate in the best optional location or add a cysteine in synthetic process, followed by mercapto-reactive PEG derivative (such as PEGylated maleimido amine) binding to cysteine. However, i) this method changes the drug structure, causing uncertainty of drug efficacy and toxicity; ii) the introduction of cysteine may affect the stability of protein since the cysteine can be easily oxidized. Other approaches regard amino group of lysine as a PEGylation site, replacing lysine with alkaline arginine except the specific one need to react. This approach also significantly affects efficacy and toxicity of the medicine due to structural changes. The inventor of the present application demonstrated that such structural change had significantly changed the effect of therapeutic peptide by experiments.

The second is to modify the N-terminal amino group using polyethylene glycol propionaldehyde derivative in acidic conditions. Some researchers have found that the N-terminal amino group's pKa is less than the amino group of lysine residues, i.e. stronger alkaline nature. Thus, the PEGylation with amino group of lysine need to be conducted in alkaline condition (pH7-8) while N-terminal amino group to be conducted in acidic conditions (pH 5-6) without amino groups of lysine reacted. Accordingly, some researchers proposed site-specific PEGylation on the N-terminal amino groups under acidic conditions. However, this approach has two drawbacks, firstly, it cannot be applied if the active site is on the N-terminus; secondly, given a molar ratio of PEG derivative up to 5 or 10, the recovery is only 50%-60%, resulting in high cost and impossibility for production.

Having no cysteine, PEG maleimide cannot be used for modifying Exendin-4 analogue using site-specific PEGylation; and the N-terminus active site (see British Journal of Pharmacology, 2003, 140, 339-346), cannot support N-terminal site-specific PEGylation using polyethylene glycol propionaldehyde.

Exendin-4 analogues contain four lysines at most, in total 5 potential reaction sites if including the N-terminal amino group. Hence, when polyethylene glycol succinimidyl derivative is used to modify Exendin-4 analogs' amino groups on the side chain, the process will inevitably produce a mixture of multi-PEGylated and mono-PEGylated products. Among the mixture, only one compound is the best active target product.

An exemplary embodiment of a method of the prior art for preparing PEGylated exendin-4 conjugates is disclosed in WO 2010/107256 A2.

Youn et al. proposed a method for protecting the amino groups, which are undesirable to be involved in the reaction, using the Fmoc (9-fluorenyl methoxycarbonyl) (Biocomjugate Chem, 2007,18,500-506). However, this approach has a significant disadvantage, that is, Fmoc protecting group is very easy to fall off in the alkaline environment. In fact, many experiments on peptide synthesis use a base (20% piperidine) to remove the Fmoc protecting groups. PEGylation of the amino group in the Exendin-4 analogues can occur in a certain alkaline condition. These contradictory situations cause Fmoc is very easy to fall off when peptides, which is protected by Fmoc, are PEGylation modified in alkaline conditions, and inevitably a significant amount of multi-PEGylated subproducts are produced.

Therefore, it is necessary to provide a process for preparing site-specific mono-PEGylated Exendin-4 analogs.

### Detailed description

The present invention is directed to a method for preparing PEGylated Exendin analogs as defined in the claims as well as to a PEGylated Exendin analog as defined in the claims. Any other disclosure in the present description not claimed, regardless of whether it is indicated as preferred or exemplified, does not form part of the present invention.

The present invention is to provide a method for preparing site-specific mono-PEGylated Exendin analogs and the site-specific mono-PEGylated Exendin analogs prepared by the method mentioned.

To achieve the mentioned object of the invention, the present invention provides a process for preparing a site-specific mono-PEGylated Exendin analog, which comprising the steps as follows:
(1) The synthesis of peptide raw materials of Exendin-4 analogues.
   Some Lysine residues of the peptide are protected by protecting group of Dde, wherein Dde is N-α-1-(4,4-dimethyl-2,6-dioxo-cyclohexyl-ylidene);
(2) The reaction between the peptide material and the polyethylene glycol reagent is carried out in alkaline organic solvent, making Lys residues without Dde binding the polyethylene glycol group;
(3) Remove the protecting group of the product produced from step (2). After separation and purification, PEGylated Exendin analog is obtained.

The method disclosed in present invention utilizes Dde as protecting group which has stronger resistance to alkaline condition than Fmoc, thereby avoiding multi-PEGylation and a variety of subproducts due to instability (falling off) of Fmoc. Hence, the subproducts are greatly reduced, and that makes the large-scale preparation possible.

In line with disclosed method, the peptide raw material should have at least one locus on the Lys residue without protected by Dde for allowing connecting the polyethylene glycol group; preferably, only one locus in the Lys residue without protected by Dde for facilitating preparation of a site-specific mono-PEGylated Exendin analog.

In accordance with disclosed method, the N-terminus of the peptide can be protected by a Dde protecting group or Fmoc protecting group. From the aspect of pharmaceutical purity, N-terminus with Dde protected is better; however in terms of the cost, the N-terminal with Fmoc protected has much possibility to be selected as the reaction demonstrated that the Fmoc protected N-terminal does not bring too many multi-PEGylated Exendin analogs.

According to the method of the invention, the specific peptide raw material may has the following structure: wherein, X is Fmoc or Dde; Z is Leu or Ile; Y₁-Y₄ is Lys or (Dde) Lys, and at least one of Y₁-Y₄ is Lys; preferably, among Y₁-Y₄, merely one of Y₂-Y₄ is Lys, the rest are (Dde) Lys; more preferably, Y₂ is Lys, Y₁, Y₃ and Y₄ are (Dde) Lys.

The method of present invention is appropriate to prepare a mono-PEGylated Exendin analog. The present invention uses peptide having four Lys residues for preparing mono-PEGylated Exendin analogues as the structure of Exendin analogs with four Lys residues is relatively complex.

According to the production method of the present invention, molecular weight (MW) of PEG derivative (PEGylation derivative) is 20,000-60,000 Da. Furthermore, polyethylene glycol having branch structures disclosed in CN 101125207A can be used in line with the method of invention.

For example, one embodiment of the present invention is as follows:
Preparing and purifying site-specific mono-PEGylated Exendin-4 analogue under the following procedures:
1. Synthesis of Exendin-4 analogue with protective groups
   If PEGylation takes place at the N-terminal amino group, Exendin-4 analogue of the following structure with protective groups is synthesized: If PEGylation takes place at the side-chain amino group of Lys12, Exendin-4 analogue of the following structure with protective groups is synthesized: If PEGylation takes place at the side-chain amino group of Lys²⁰, Exendin-4 analogue of the following structure with protective groups is synthesized: If PEGylation takes place at the side-chain amino group of Lys²⁷, Exendin-4 analogue of the following structure with protective groups is synthesized: If PEGylation takes place at the side-chain amino group of Lys⁴⁰, Exendin-4 analogue of the following structure with protective groups is synthesized:
2. The Exendin-4 analogs having protective groups and PEG derivative having a MW of 20,000-60,000 Da with a certain molar ratio (preferably 40KD Y-type PEG-NHS ester) are solved in an appropriate amount of organic solvent (preferably DMSO). After completely dissolved, an organic base that is non-reactive with PEG derivative is added to achieve an alkaline environment. The optional reagents are triethylamine (TEA), diisopropylethylamine (DIEA), 4-dimethylaminopyridine (DMAP), 2,4,6-trimethyl pyridine (colidine), lutidine (lutidine), pyridine (pyridine), etc.
3. The PEGylation reaction is carried out by preserving the solution system at a certain temperature (not exceeding 40°C) for some time. Subsequently, sufficient amounts of reagents (preferably hydrazine hydrate) are added to remove Fmoc and Dde protecting groups. All protecting groups are removed at a certain temperature (less than 40°C) for some time.
4. The final reaction solution is diluted 10-fold with pure water and pH is adjusted to 5.0-6.0 immediately using HCL or acetic acid to ensure stability of the sample. Then SOURCE 30RPC filler and water containing 20 mM acetic acid: acetonitrile or water: ethanol system are utilized to achieve the isolation and purification of the target PEGylated Exendin-4 analogue using linear gradient elution method.
5. Furthermore, purify the target compounds (containing some organic solvent, acetonitrile or ethanol) obtained from last step using a cation exchange resin, 10mM citrate buffer salt, 1.5M NaCl, to remove the organic solvent with gradient elution method.
6. Conduct ultrafiltration for the PEGylated Exendin-4 analogue obtained from step 5 using 10KD ultrafilter film. Molecular sieve chromatography is used for desalting with pure water. After lyophilized the resulting aqueous solution of PEGylated Exendin-4 analogues, the PEGylated Exendin-4 analogue raw materials are obtained.

To achieve the object of the present invention, PEGylated Exendin analogue prepared by the above-mentioned method is provided.

To achieve the purpose of the present invention, the present invention also provides a PEGylated Exendin analog, wherein the Exendin analog has the following sequence: wherein, Z is Leu (SEQ ID No. 1) or Ile (SEQ ID No.2), and one Lys residue's amino group is connected to polyethylene glycol.

Preferably, in the above-described sequence, the amino group of Lys²⁰ residue or Lys²⁷ residue is connected to polyethylene glycol.

It is also disclosed the usage on treatment of diabetes or obesity using the PEGylated Exedin analogues.

According to the method of the present invention Dde as a protecting group of higher stability is used to avoid multi-PEGylation brought about by the unstable Fmoc, achieving a low cost and a high recovery of PEGylated Exedin analogue with a low molar ratio of reactants. PEGylated Exedin analogues of the present invention are site-specific mono-PEGylated Exedin analogs, and have few by-products, which helping avoid various side effects caused by the by-products.

For a more detailed description of the invention, the accompanying drawings are used to describe the specific embodiment by means of specific implement methods.

### Description of figures

Figure 1 is a MALDI-TOF mass spectrum of site-specific protected Exendin-4 analogue;
Figure 2 is HPLC chromatograms before and after PEGylation of the site-specific protected Exendin-4 analogue;
Figure 3 is a SOURCE purification chromatogram of PEGylated Exendin-4 analogue;
Figure 4 is a SP cation exchange purification chromatogram of PEGylated Exendin-4 analogue;
Figure 5 is a molecular sieve desalination chromatogram of PEGylated Exendin-4 analogue;
Figure 6 shows the effect of mono-PEGylated HYBR-003-PEG on C57 BL/6 mice intraperitoneal glucose tolerance test (n = 8), wherein, a P <0.01 V.S blank, b P <0.01 V.S control.

### Specific implement methods

### Example 1

(1) Amino acid monomers used in the synthesis The abbreviation of the above: Fmoc 9-fluorenyl-methoxycarbonyl; Boc tert-butoxycarbonyl group; Trt trityl; OtBu tert-butoxy; tBu tert-butyl; Dde N-[1-(4,4-dimethyl-2,6- dioxo-cyclohexylene)
(2) Reagents: N,N-diisopropylethylamine, diisopropyl carbodiimide (DIC), N, N-dimethylformamide (DMF), dichloromethane, hexahydropyridine, 1-hydroxybenzotriazole triazole, Rink Amide resin, ninhydrin, methanol, triisopropylsilane, trifluoroacetic acid.
(3) Operation

### A. synthesis: as illustrated by the scale of 0.25mmol, the sequence was synthesized from C- to N-terminal in a reactor in the presence of 0.5g Rink Amide resin and 1mmol amino acid which had been activated with DIC/HOBt method. Reaction was carried out at room temperature of 25 °C, and the process was operated as follows:

1. The protecting group Fmoc was removed by using 20% piperidine DMF solution, 10 minutes each process;
2. Wash three times using 10mL DMF, drain;
3. Protected amino acid (1mmol) and HOBt (1mmol) were dissolved in 10mLDMF, DIC (1mmol) was added for activating for 10 minutes;
4. The activated amino acid solution was added to the reactor, shaking for 1 hour;
5. Wash three times with DMF, and drain;
6. If the ninhydrin reaction is negative, it should be proceeded to repeat steps 1-5;

If positive, repeating steps 3-5.

After completion of synthesis of peptide chain, wash the resin with methanol, and dry it.

### B. Remove protecting group and cut off excessive peptide

1g of peptide-attached resin was added in a reactor, and then lysis solution (ratio: 2ml of anisole, 2ml of methanol, 2ml of triisopropylsilane and 6ml of trifluoroacetic acid) was added.

The sample was shaken for 2 hours at room temperature. After the filtering, the filtrate was collected. The resin was washed with a small amount of acetic acid. The collected samples were combined and concentrated. Diethyl ether was added to precipitate, after filtering the precipitate, the sample was washed with a little amount of diethyl ether, and then the crude product was obtained..

### C. The Samples were separated and purified by preparative HPLC, and lyophilized.

The resulting crude product was dissolved in a small amount of 10% acetic acid solution, loaded on the column, purified by the preparative HPLC, and then lyophilized. The resulting peptides were proved to be the required compound by mass spectrometry.

Chromatogram column: Boston C18, 5um, 100A, wavelength of 214nm, Waters preparative HPLC

Gradient: 10% 0.05% TFA/CAN-45% 0.05% TFA/CAN 20min, 45% 0.05% TFA / CAN 10min.

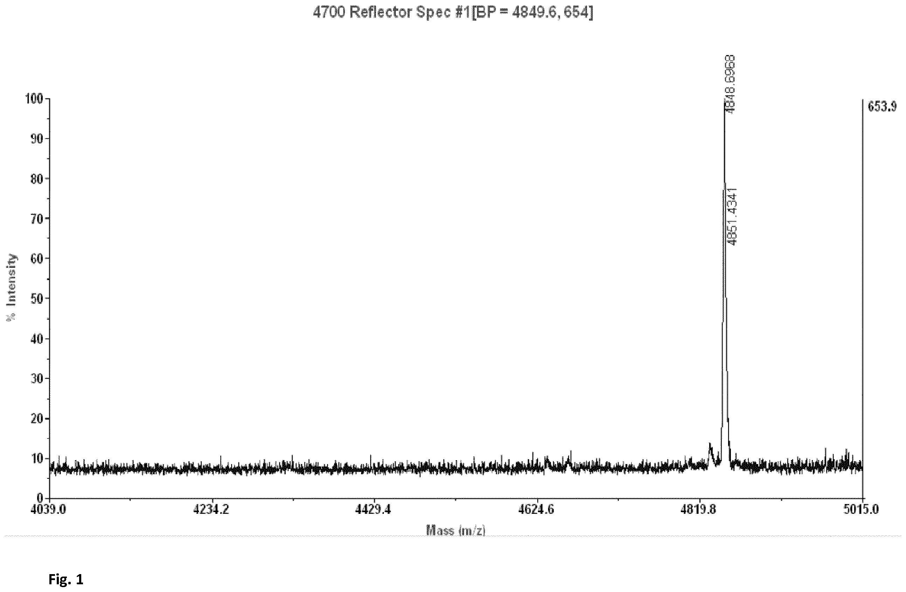

Figure 1 is MALDI-TOF mass spectrum of site-specific protected Exendin-4 analogue

### Example 2

### Method for PEGylation of site-specific protected Exendin-4 analogue

The present embodiment used conventional amino PEG derivatives such as (SC-PEG, SS-PEG, NHS-PEG, etc.) to bind and modify the exclusive free side-chain amino group which is able to be PEGylated on the Exendin-4 analogue, wherein, the PEG derivative is preferably selected from 40KD Y type NHS-PEG and site-specific protected Exendin-4 analogue is:

2g site-specific protected Exendin-4 analogue and 26g 40KD Y-type NHS-PEG (with a molar ratio versus peptide of about 1.5:1) were dissolved in 400mL DMSO, then stirred at 40 °C continuously. After completely dissolved, 200uL (0.05%) of triethylamine (TEA) was added to activate the PEGylation reaction at the temperature of 40°C and PEGylation was substantially completed (90% plus, calculated by protected Exendin-4 analogue) by stirring for 2h. Then, 8ml (2%) of hydrazine was added at 40 °C and the solution was stirred for 1h to remove protecting groups until the reaction finishes. The final reaction solution was diluted 10-fold using pure water and immediately adjusted pH to 6.0 using 6M HCL, and then stored refrigerated at 4 °C.

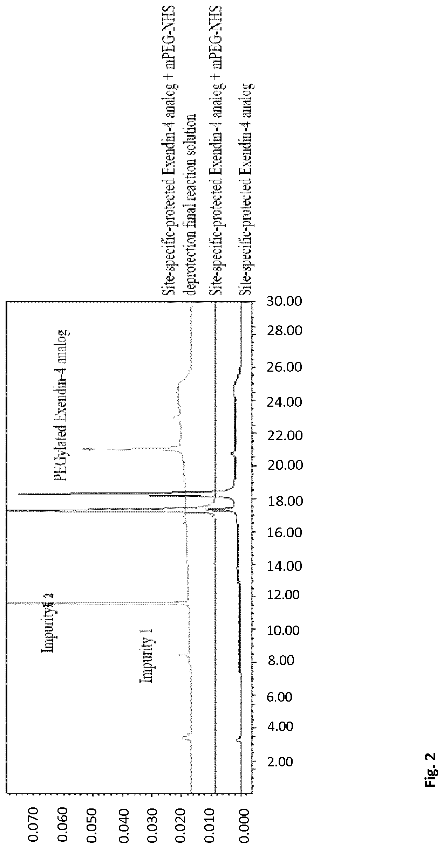

Figure 2 is HPLC chromatograms before and after PEGylation of site-specific protected Exendin-4 analogue.

### Example 3

### Method for isolation and purification of PEGylated Exendin-4 analogue

Pure PEGylated Exendin-4 analogue raw material can be obtained by reverse-phase HPLC, ion exchange, ultrafiltration, molecular sieve and lyophilization of the final reaction solution of the site-specific protected Exendin-4 analogues.

### A. Purification via SOURCE reverse phase HPLC

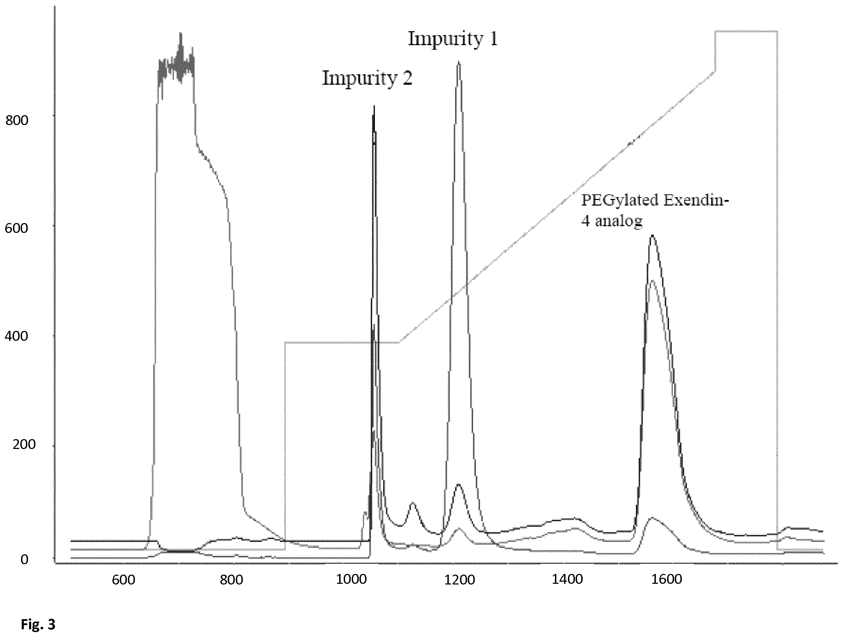

Mobile phase: phase A: 20mM HAc, 5% acetonitrile; phase B: 20mM HAc, 50% acetonitrile
Column: GE Fineline Pilot 35
Filler: SOURCE 30RPC 175mL
Flow rate: 30mL/min
Elution gradient: after delivering the sample, balance for 2 column volumes, 0-30% 5min, 30% -100% 5min
Figure 3 is SOURCE purification chromatogram of PEGylated Exendin-4 analogue.

### B. SP cation exchange purification

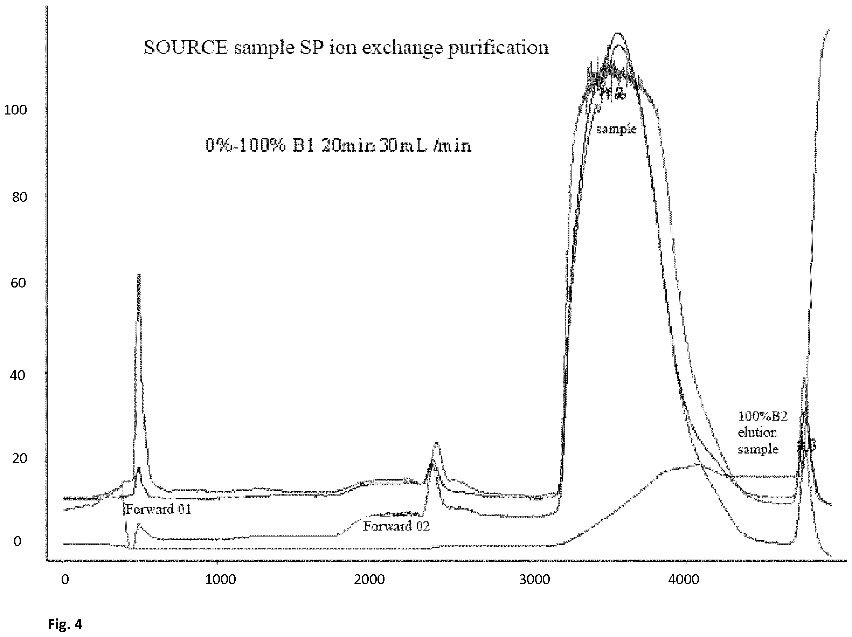

Mobile phase: phase A: 10mM pH3.5 CBS
Phase B1: 10mM pH3.5 CBS +0.15 M NaCL
Phase B2: 10mM pH3.5 CBS +1.5 M NaCL
Chromatographic column: GE XK 50
Filler: SP sepharose Fast Flow 600mL
Flow rate: 30mL/min
Gradient elution: 0% -100% phase B1 20min, 100% phase B1 - phase B2 100% 0min
Figure 4 is SP cation exchange purification chromatogram of PEGylated Exendin-4 analogue.

### C. Ultrafiltration

Device: 10KD PALL shear ultrafiltration
Pre-filter loading volume was 2400mL and 400mL after filtration
The ultrafiltration (1000mL-400mL) was repeated 3 times.

### D. G25 desalting

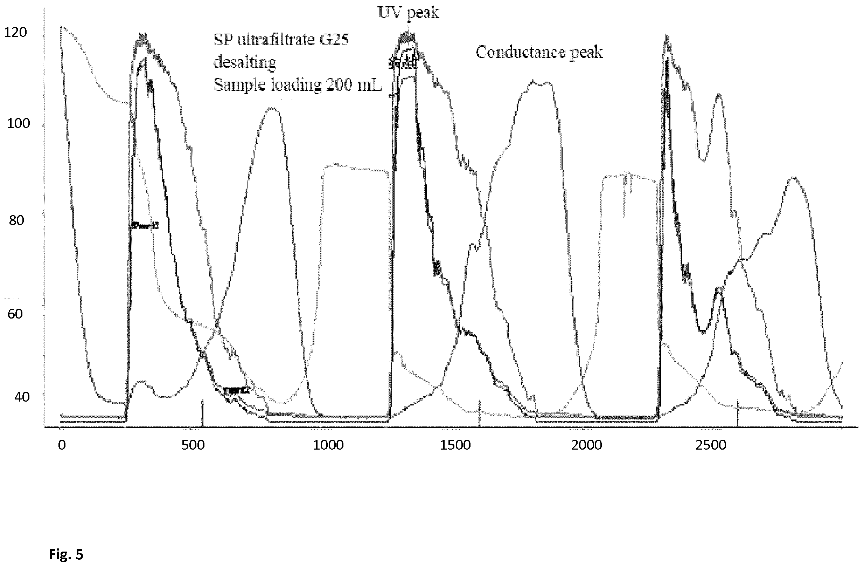

Mobile phase: water
Chromatographic column: GE XK 50
Filler: G25; 900mL
Flow rate: 30mL/min,
Loading quantity of sample: 200mL
Figure 5 is a molecular sieve desalination chromatogram of PEGylated Exendin-4 analogue.

### E. Lyophilization

Co-melting point of PEGylated Exendin-4 analogue pure water solution is about -5 °C, so the first lyophilization temperature is set at -10 °C, and the second at 5 °C. Other parameters (freeze time and the oven temperature, etc.) are set in accordance with the amount of the sample, freeze dryer performance and specific climatic conditions.

### Example 4

### Intraperitoneal glucose tolerance test in normal C57 mice (IPTT)

### (1) Materials

Animals: C57 mice were purchased from Shanghai SLACCAS Laboratory Animal Co., SPF level. Animals were raised in temporary animal house of the company, CL-class. Quantity: 60, Gender: Male

Reagents: Exendin-4 analogue (0.125 ug / ml); PEGylated Exendin-4 analogue (3.125 ug/ml, calculated by bare peptide); glucose kit; 20% glucose injection; and saline injection.

### (2) Experimental methods

Medication Administration Team: each mouse was administrated with PEGylated Exendin-4 analogue (3.125 ug / ml, calculated by bare peptide); blank group in a dosage of 0.2mL/20g; each mouse was injected with normal saline in a dosage of 0.2mL/20g; control group: each mouse was administrated with Exendin-4 analogue (0.125 ug/ml) in a dosage of 0.2mL/20g. Blood glucose load was achieved by intraperitoneal injection of 20% glucose solution 0.2ml/20g b.w. half an hour before testing the blood glucose. 24h and 72h later after the administration, the hypoglycemic effect duration of the tested animals was observed.

### (3) Experimental results

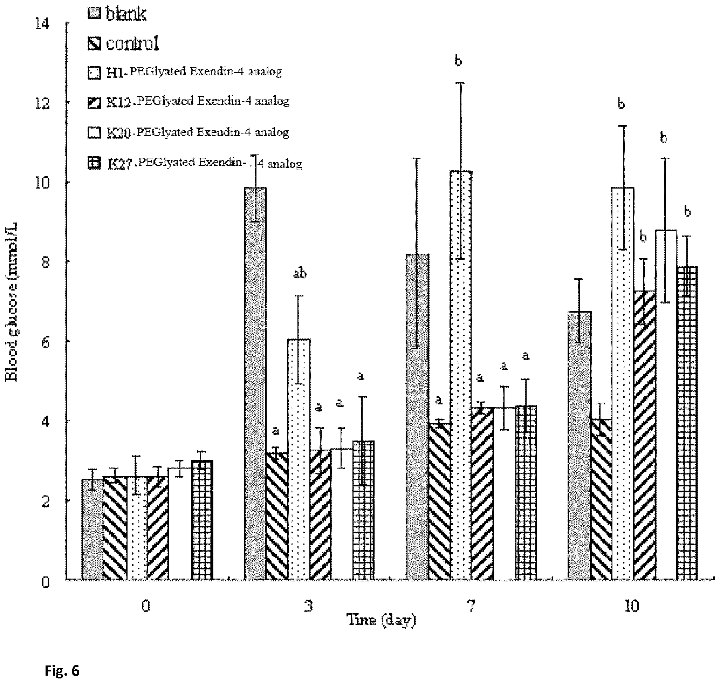

Figure 6 shows hypoglycemic effect durations of the individual PEGylated Exendin-4 analogues.

## Claims

1. A method for preparing PEGylated Exendin analogs, including the steps as follows:
(1) Synthesize peptide raw materials of Exendin analogues wherein one or more of the Lysine residues of the peptide have been protected by Dde, wherein Dde is N-α-1-(4,4-dimethyl-6-dioxo-cyclohexylene) and wherein said peptide material has the following structure: wherein X is Fmoc or Dde; Z is Leu or Ile; Y₁-Y₄ is Lys or (Dde) Lys, and at least one of Y₁-Y₄ is Lys;
(2) The peptide materials mentioned in step (1) react with the activated PEG in alkaline organic solvent, allowing Lys residues without Dde protecting group to bind the polyethylene glycol group;
(3) Remove the protecting groups of the product produced from step (2), isolate and purify the product, obtaining the PEGylated Exendin analogs.

2. The method according to claim 1, wherein only one of Y₁-Y₄ is Lys without protection by Dde.

3. The method of claim 1, wherein the N-terminus of the peptide material is protected by Dde or Fmoc and Fmoc is 9-fluorenyl-methoxycarbonyl.

4. The method of claim 1, wherein in Y₁-Y₄ only one of Y₂-Y₄ is Lys, the rest are (Dde) Lys.

5. The method of claim 4, wherein Y₂ is Lys, Y₁, Y₃ and Y₄ are (Dde) Lys.

6. PEGylated Exendin analogue prepared according to one of claims 1 to 5, wherein said PEGylated Exendin analogue has the following sequence structure: Wherein, Z is Leu or Ile, and in said structure the amino group of one Lys residue is connected to polyethylene glycol.

## Patentansprüche

1. Verfahren zur Herstellung von PEGylierten Exendin-Analoga, umfassend die Schritte wie folgt:
(1) Synthetisieren der Peptid-Ausgangsmaterialien der Exendin-Analoga, wobei ein oder mehrere der Lysinreste des Peptids mit Dde geschützt wurden, wobei Dde N-α-1-(4,4-Dimethyl-6-dioxo-cyclohexylen) ist und wobei das Peptid-Material die folgende Struktur aufweist: wobei X Fmoc oder Dde ist; Z Leu oder Ile ist; Y₁ bis Y₄ Lys oder (Dde)Lys sind, und mindestens eines von Y₁ bis Y₄ Lys ist;
(2) die in Schritt (1) erwähnten Peptid-Materialien reagieren mit dem aktivierten PEG in einem alkalischen organischen Lösungsmittel, indem Lys-Reste ohne Dde-Schutzgruppe die Polyethylenglykolgruppe binden können;
(3) Entfernen der Schutzgruppen von dem gebildeten Produkt aus Schritt (2), Isolieren und Reinigen des Produkts, wodurch die PEGylierten Exendin-Analoga erhalten werden.

2. Verfahren nach Anspruch 1, wobei nur eines von Y₁ bis Y₄ Lys ohne Schutz durch Dde ist.

3. Verfahren nach Anspruch 1, wobei der N-Terminus des Peptid-Materials mit Dde oder Fmoc geschützt wird und Fmoc 9-Fluorenylmethoxycarbonyl ist.

4. Verfahren nach Anspruch 1, wobei bei Y₁ bis Y₄ nur eines von Y₂ bis Y₄ Lys ist, der Rest (Dde)Lys sind.

5. Verfahren nach Anspruch 4, wobei Y₂ Lys ist, Y₁, Y₃ und Y₄ (Dde)Lys sind.

6. PEGylierte Exendin-Analoga, hergestellt nach einem der Ansprüche 1 bis 5, wobei die PEGylierten Exendin-Analoga die folgende Sequenzstruktur aufweisen: wobei Z Leu oder Ile ist, und in der Struktur die Aminogruppe eines Lys-Restes mit Polyethylenglykol verbunden ist.

## Revendications

1. Procédé pour préparer des analogues d'exendine PÉGylés, comprenant les étapes suivantes :
(1) Matières premières peptidiques synthétisées d'analogues d'exendine, un ou plusieurs résidus de lysine du peptide ayant été protégés par Dde, Dde étant N-α-1-(4,4-diméthyl-6-dioxo-cyclohexylène) et ladite matière peptidique ayant la structure suivante : est Fmoc ou Dde ; Z est Leu ou Ile ; Y₁-Y₄ est Lys ou (Dde) Lys, et au moins un élément parmi Y₁-Y₄ est Lys ;
(2) Les matières peptidiques mentionnées à l'étape (1) réagissent avec le PEG activé dans un solvant organique alcalin, permettant aux résidus de Lys sans groupe protecteur Dde de se lier avec le groupe polyéthylène glycol ;
(3) Enlever les groupes protecteurs du produit produit lors de l'étape (2), isoler et purifier le produit, obtenir les analogues d'exendine PÉGylés.

2. Procédé selon la revendication 1, un élément seulement parmi Y₁-Y₄ étant Lys sans protection par Dde.

3. Procédé selon la revendication 1, le terme N du matériau peptidique étant protégé par Dde ou Fmoc et Fmoc étant 9-fluorényl-méthoxycarbonyle.

4. Procédé selon la revendication 1, uniquement un élément parmi Y₂-Y₄ étant Lys dans Y₁-Y₄, le reste étant (Dde) Lys.

5. Procédé selon la revendication 4, Y₂ étant Lys, Y₁, Y₃ et Y₄ étant (Dde) Lys.

6. Analogue d'exendine PÉGylé préparé selon l'une des revendications 1 à 5, l'analogue d'exendine PÉGylé ayant la structure de séquence suivante : Z étant Leu ou Ile, et le groupe amine d'un résidu de Lys étant lié au polyéthylène glycol dans ladite structure.


### SEQUENCE LISTING

<110> Shanghai Huayi Bio-Lab Co., Ltd. YUE, Peng
<120> SITE-DIRECTED MONO-SUBSTITUTED PEGYLATED EXENDIN ANALOG AND PREPARATION METHOD THEREFOR
<130> 057783.8012.US00
<150> PCT/CN2012/71910
   <151> 2012-03-05
<150> CN 201110078314.X
   <151> 2011-03-30
<160> 2
<170> PatentIn version 3.5
<210> 1
   <211> 40
   <212> PRT
   <213> Artificial Sequence
<220>
   <223> Exendin-4 analog
<400> 1
   His Gly Glu Gly Thr Phe Thr Ser Asp Leu Ser Lys Gln Leu Glu Glu
   1               5                   10                  15
   Glu Ala Val Lys Leu Phe Ile Glu Trp Leu Lys Asn Gly Gly Pro Ser
               20                  25                  30
   Ser Gly Ala Pro Pro Pro Ser Lys
           35                  40
<210> 2
   <211> 40
   <212> PRT
   <213> Artificial Sequence
<220>
   <223> Exendin-4 analog
<400> 2
   His Gly Glu Gly Thr Phe Thr Ser Asp Leu Ser Lys Gln Ile Glu Glu
   1               5                   10                  15
   Glu Ala Val Lys Leu Phe Ile Glu Trp Leu Lys Asn Gly Gly Pro Ser
               20                  25                  30
   Ser Gly Ala Pro Pro Pro Ser Lys
           35                  40